Europäisches Patentamt

European Patent Office

Office européen des brevets

(19)

(11) Publication number: **0 178 435**
**A2**

# (12) EUROPEAN PATENT APPLICATION

(21) Application number: 85110967.8

(22) Date of filing: 30.08.85

(51) Int. Cl.⁴: **C 07 H 21/04**
**C 12 N 15/00, C 12 Q 1/70**

(30) Priority: 31.08.84 US 646592
28.01.85 US 695587

(43) Date of publication of application:
23.04.86 Bulletin 86/17

(84) Designated Contracting States:
AT BE CH DE FR GB IT LI LU NL SE

(71) Applicant: Research Corporation
Suite 853, 25 Broadway
New York New York(US)

(72) Inventor: Bishop, Polly Roy
3339 Brookwood Road
Birmingham Alabama(US)

(74) Representative: Patentanwälte Grünecker, Kinkeldey,
Stockmair & Partner
Maximilianstrasse 58
D-8000 München 22(DE)

(54) cDNA probe for the detection of bluetongue virus.

(57) The genes of the bluetongue virus (BTV) serotype 17 have been cloned into pBR322 by tailing both strands by the double-stranded RNA with poly (A), transcribing them with reverse transcriptase using an oligo dT primer, hybridizing the cDNA products and completing them into duplex structures using the Klenow fragment of *E. Coli* DNA polymerase. After cloning the double-stranded cDNA molecules into pBR322 the complete sequence of the cloned L3 gene was determined. The clone is 2,772 nucleotides long (1.78 × 10⁶ daltons) excluding the 3′ polyadenylic acid sequence and has an open reading frame which codes for a protein of some 901 amino acids (103, 412 daltons). This clone can hybridize L3 RNA segments of a variety BTV serotypes but not the equivalent RNA segment of epizootic haemorrhagic disease of deer (EHD) virus, a bluetongue related orbivirus.

Fig. 3

EP 0 178 435 A2

## DNA PROBE FOR THE DETECTION
## OF BLUETONGUE VIRUS

This application is a continuation-in-part of my copending U.S. application, Serial No. 646,592, filed August 31, 1984.

This invention was made with support in part by U.S. Department of Agriculture Grant 82 CRSR-2-1032 and IRS Award A107150 from The National Institute of Allergy and Infection Disease.

This invention relates to the field of molecular diagnostics. More specifically, the invention relates to a gene from Bluetongue virus, a method for synthesizing such a gene, and the use of such a gene for the detection of Bluetongue virus and for the production of viral protein.

Bluetongue is an arthropod-borne disease of sheep and cattle. Bluetongue virus (BTV), the etiologic agent of the disease, is classified as a member of the Reoviridae family of viruses. These so-called Reo viruses derive their name from the acronym Respiratory-Enteric-Orphan; the latter denomination relates to the lack of association with any disease in humans. The major criterion for inclusion in the family is the possession of a genome consisting of 10, 11 or 12 segments of double-stranded RNA (ds RNA). More specifically, BTV is classified as a member of the genus orbivirus owing to its possession of ring-shaped capsomeres. The genome of BTV is composed of ten segments of double-stranded RNA which are encapsulated by a two-layer protein coat. The proteins have been shown to be antigenic and form the basis of serological typing of this group of viruses. The four serotypes common to the United States are BTV-10, 11, 13 and 17. The serotypes have been shown to be genetically related; that is to say each has comparable RNA fingerprints and each can reassort its viral RNA species (Sugiyama, K. et al., Virology 114: 210-217, (1981)).

The ten RNA segments can be separated electrophoretically on agarose gels (Sugiyama, K. Amer. J. Epidemiol 115: 332-347 (1981). In the gel system employed, the ten segments appear as bands arranged in linear fashion from top to bottom of the gel. The three uppermost bands are denominated as L1, L2, L3 based on their relatively large molecular weight. The next three species are denominated as M1, M2 and M3 and the the latter four species are denominated as S1, S2, S3 and S4. Owing in part to their smaller molecular weight, it is possible for two of the S1-S4 species to comigrate yielding a single thickened band rather than discrete entities. For example, in the serotype 17, RNA segments S1 and S2 comigrate. In certain cases the function of the protein encoded by the respective RNA species is known. As mentioned above, the genetic information of the virus is encapsulated within a two-layer protein coat; in the case of the serotype 17, RNA species L2 and M2 encode proteins which comprise the outermost protein coat whereas RNA species L3, L1, M1 and S1 encode components of the inner protein coat.

In addition to the four BTV serotypes common to the United States, these and other BTV serotypes are known to be endemic to other countries. For example, BTV 1-15 are known to occur in South Africa, BTV-16 is common in W. Pakistan and BTV-20 and -21 are found in Australia.

As mentioned above Bluetongue is an arthropod-borne disease of sheep and cattle. In sheep, the disease is characterized by high morbidity and variable but significant mortality. In cattle, mortality is usually lower, although various complications of the disease occur. The disease affects not only the reproduction of sheep and cattle but also, for recovered animals, loss of weight, wool and milk production. Cattle can become persistently infected and are capable of transmitting the virus to the fetus. Semen can also be a reservoir of the virus. In light of these observations, bluetongue virus is of considerable concern to those involved in the export and import of animals and their germ plasm.

Serologic testing is used extensively to detect the presence of serum specific antibodies against BTV. Unfortunately, the serological procedures have limitations. Either cross-reaction occurs with other members of the orbivirus group including epizootic hemorrhagic disease virus (EHDV), Eubenangee (EUB), and Palyam (PAL) viruses (Moore, D.L., Amer. J. Vet. Res. 35:1109(1974)). For EHDV cross-reactions are of particular concern. Another drawback is that the current serologic tests are not sensitive or accurate enough (Bowen, R.A. et al., Proc. 8th Nat'l. Animal Breeders Tech. Conf., pp 79-83 (1980)). The recent development of recombinant DNA techniques and methods for analyzing the intracellular localization of specific genes can be exploited to develop sensitive and specific nucleic acid probes for diagnosis purposes.

It is an object of this invention to provide a nucleic acid-based detection system which is capable of both serotype-specific or group- specific detection of Bluetongue virus.

Brief Description of the Drawings

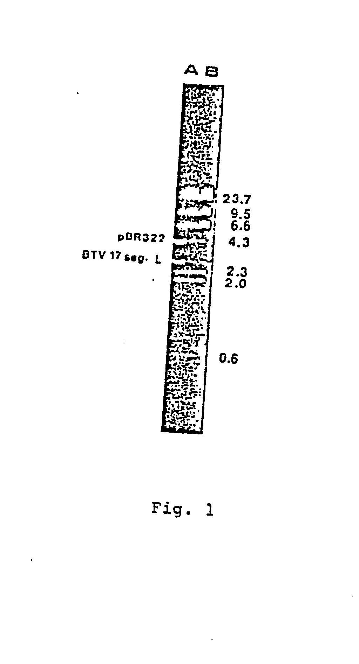

Figure 1 illustrates the migration pattern of clone 7 plasmid DNA cut with Hind III and run on a 1% agarose gel; A. Migration pattern of clone 7 DNA Hind III fragments. The position of the pBR322 vector and the BTV-17 segment 2/3 cDNA are marked on the left side of the figure, B. The <u>Hind III</u> restriction fragments of cDNA with the size of the fragments in kilobases listed at the right of the figure.

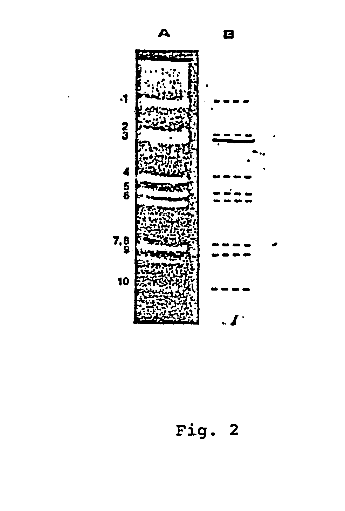

Figure 2 illustrates the nicked translation hybridization of clone 7 plasmid DNA; A. RNA migration pattern of BTV-17 genomic RNA run on an agarose gel, B. Autoradiograph of clone 7 DNA hybridized to BTV-17 segment 3 RNA. The numbers to the left of the figure represent the numbering system for BTV genomic RNA. Segments 7 and 8 comigrate in agarose gels.

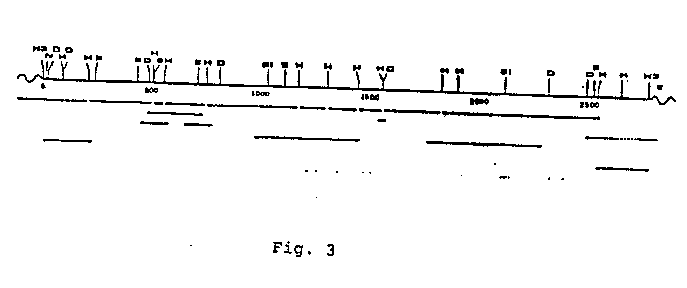

Figure 3 illustrates the sequence strategy used to determine the sequence of the cloned BTV-17 segment 3 gene. The restriction enzymes used to generate the restriction fragments are shown on the right side of the figure. The distances and directions individual strands were sequenced are shown by the solid arrows. Restriction site symbols used are: H3, <u>Hind III</u>; N, <u>Nco I</u>; D, <u>Dde I</u>; H, <u>Hinf I</u>; P, <u>Pst I</u>; B, <u>BamHI</u>; E, <u>EcoRI</u>; and Bl, <u>Bgl II</u>.

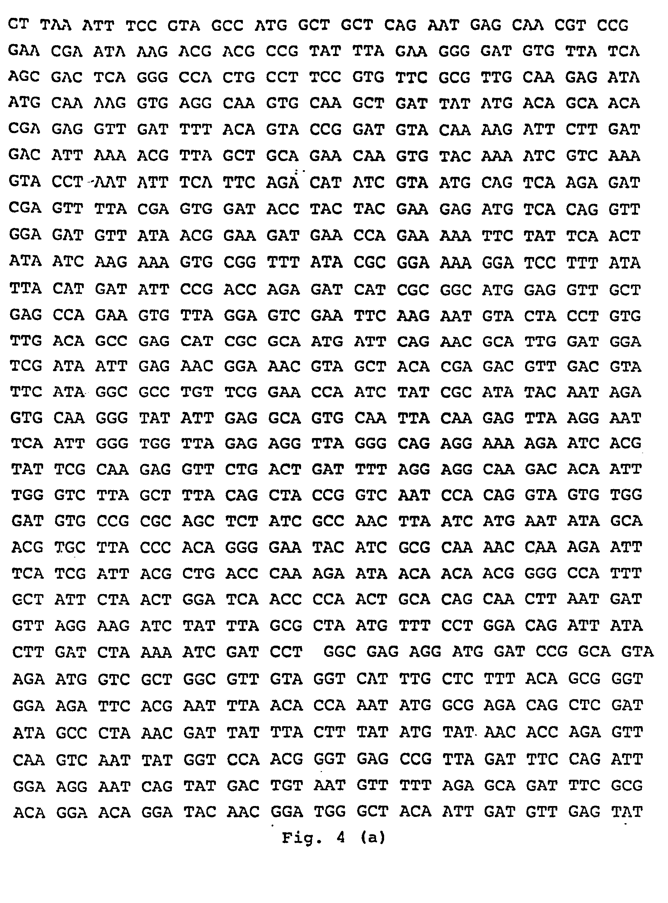

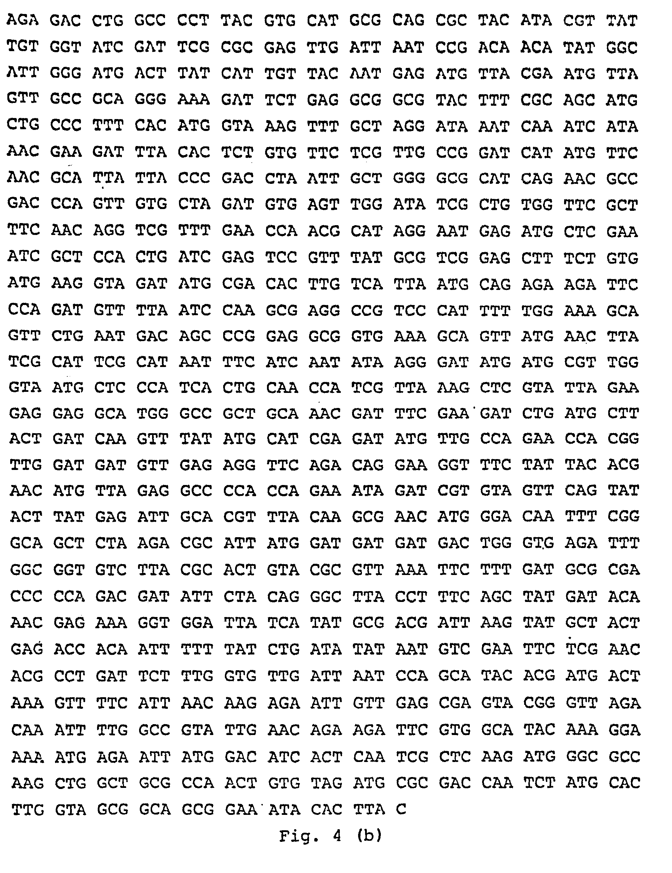

Figure 4 shows the nucleotide sequence of the cloned L3 gene of BTV-17. The open reading frame begins at position 18-20 and is terminated by the TAG codon at position 2721-2723.

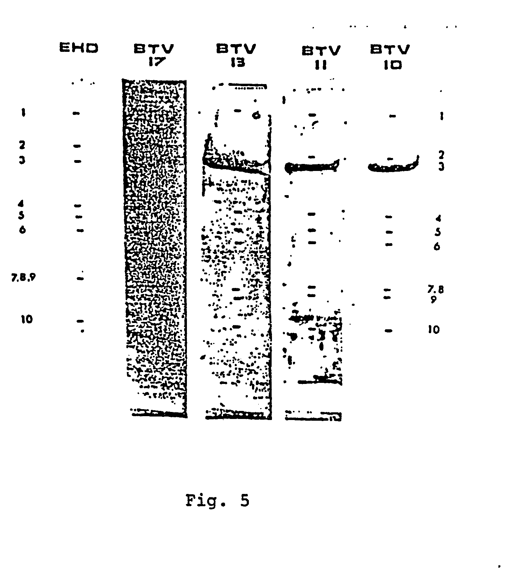

Figure 5 shows the hybridization of BTV clone 7 to BRV and EHD L3 RNA segments; autoradiograms of nick translated $^{32}$P-labelled clone 7 DNA hybridized to genomic RNAs of four U.S. BTV serotypes and EHD serotype 1, purified from infected cells. The numbers to the sides of the figure represent the numbering system for the genomic RNA segments. Segments 7 and 8 for BTV and segments 7, 8, 9 for EHD comigrate in agarose gels.

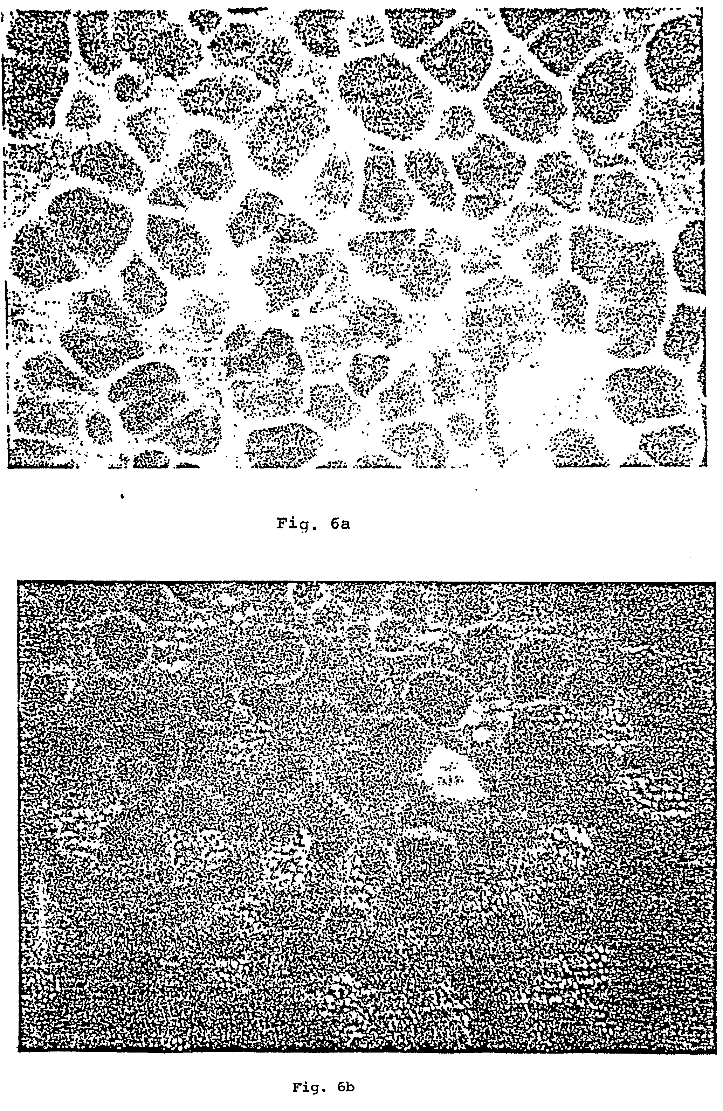

Figure 6 illustrates the detection of Bluetongue virus by *in situ* hybridization with a biotinylated BTV-17 probe DNA. 6A noninfected control cells. 6B 20 hr BTV-infected cells.

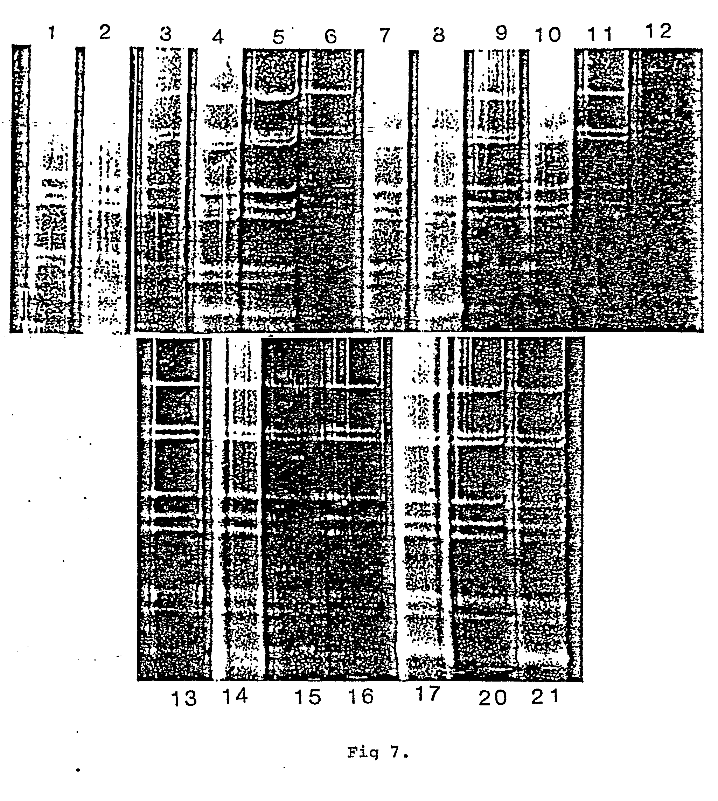

Figure 7 illustrates the resolution by gel electrophoresis of the genome RNA segments of BTV: Genome RNAs of BTV-1-15 (S. Africa), -16 (W. Pakistan), -17 (U.S.), -20 & -21 (Australia) were purified from infected BHK-21 cells and resolved in 1% Agarose gels. The RNA segments of each BTV serotype were identified by staining the gel in ethidium bromide.

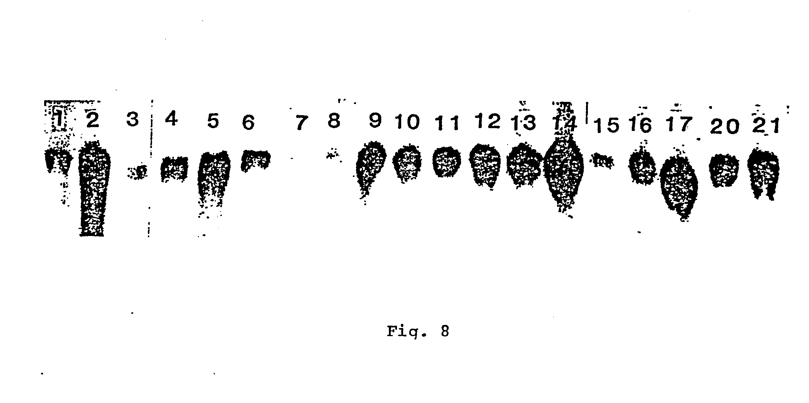

Figure 8 illustrates the hybridization by northern blotting of segment 3 DNA of US BTV-17 to corresponding RNA segments of other BT viruses: Autoradiograms of nick-translated, $^{32}$P-labelled clone DNA hybridized to genome RNAs of nineteen BTV serotypes blotted in gene-screen paper. Each BTV serotype is specified on top of the autoradiogram.

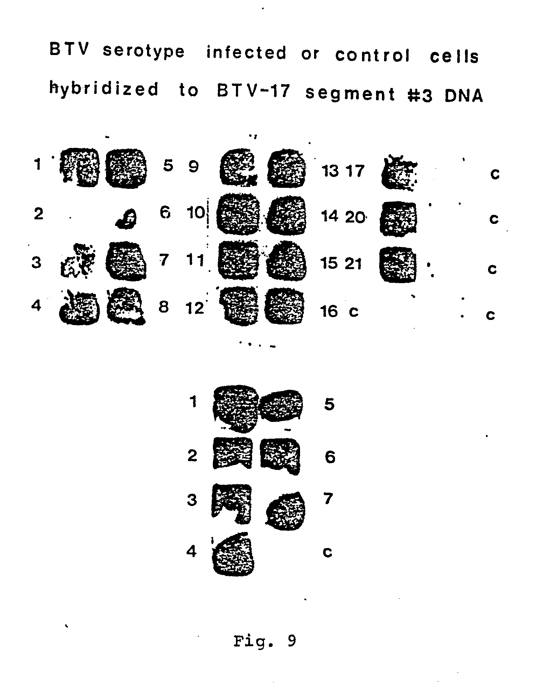

Figure 9 illustrates the *in situ* hybridization of clone DNA to viral genomes: Autoradiogram of nick-translated $^{32}$P-labelled clone DNA hybridized to monolayers containing infected or control cells. The different BTV serotypes (1-17, 20, & 21) and the control cells (c) are indicated on the side of each chamber.

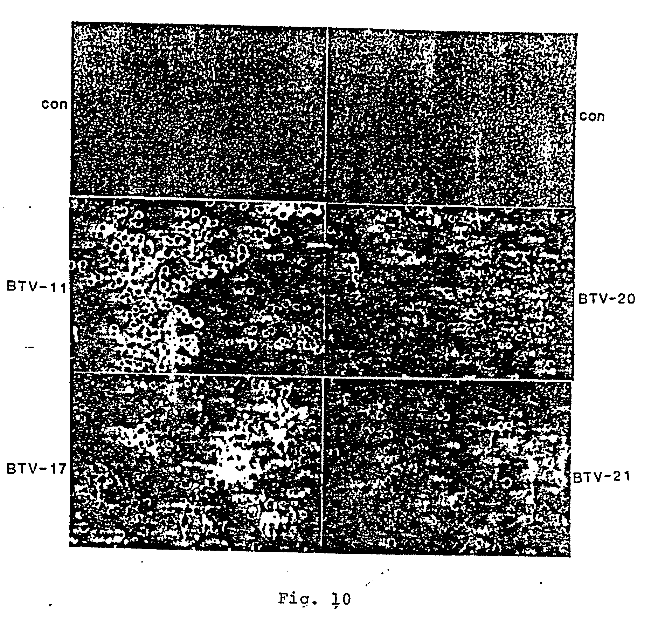

Figure 10 illustrates the fluorescence detection of the hybrids in culture cells: The nick-translated biotinylated dUTP labelled DNA clone was hybridized to viral genomes present in cells infected with either 11 or 17 or 20 or 21 in chamber slides. The hybrids were detected by fluorescence microscopy following the incubation with avidin-fluorescence complex for 30 minutes in 18°C. Two chambers containing controls, uninfected cells are indicated as "C".

This invention provides a DNA sequence characterized in that it is capable of hybridizing with a portion of the RNA genome of at least one serotype of Bluetongue virus.

In a further embodiment the invention provides a cloning vector characterized in that it comprises a DNA sequence capable of hybridizing with a portion of the RNA genome of at least one serotype of Bluetongue virus.

In yet another embodiment the invention provides a method for producing a duplex DNA sequence that is capable of hybridizing with a portion of the RNA genome of at least one serotype of Bluetongue virus which comprises:

a) isolating a double-stranded RNA species from a Bluetongue virus serotype;

b) separating the RNA strands;

c) polyadenylating the 3' ends of each RNA strand to provide RNA template strands

d) synthesizing a cDNA copy of each RNA template strand by contacting said polyadenylated RNA template strands with a reverse transcriptase in the presence of oligo dT under conditions of time and temperature sufficient for the production of cDNA;

e) removing the RNA template strands; and

f) self-annealing the cDNA products to form cDNA duplexes.

In yet another embodiment the invention provides a DNA probe useful for the detection of Bluetongue virus characterized as comprising a DNA sequence complementary to a portion of the RNA genome of said virus and capable of hybridization therewith.

0178435

In yet another embodiment the invention provides a method for detection of Bluetongue virus in a sample containing same comprising:

a) providing a probe DNA complementery to a portion of the RNA genome of said virus

b) contacting said probe with said RNA under conditions promoting the formation of DNA-RNA hybrids and

c) detecting the DNA-RNA hybrids thereby detecting the presence of the virus in said sample

In a further embodiment the invention provides a kit containing reagents useful for the detection of BTV in a sample.

In a final embodiment the invention provides a method of producing viral protein comprising culturing a host cell under conditions promoting the synthesis of viral protein, said host cell being further characterized as being transformed by an expression vector comprising a bluetongue viral DNA sequence, said sequence being in an orientation with a promoter sequence such that in the host said viral DNA is expressed to produce a bluetongue viral protein.

This invention relates to the cloning of genetic information of the Bluetongue virus. Such information is useful as a probe for the detection of viral genomes in samples as well as for the production of viral protein by means of recombinant DNA techniques.

The rationale employed for the cloning of the virus genetic information was as follows: the individual double-stranded RNAs were isolated from agarose gel following their separation by electrophoresis, the strands were separated and their 3' ends polyadenylated by reaction with poly (A) polymerase; cDNA copies were synthesized employing

0178435

oligo T as a primer and reverse transcriptase; after removal of the RNA template and the cDNA products were self-annealed; to ensure that all strands were full-length, their 3' ends were repaired using the Klenow fragment of E. coli DNA polymerase I the resulting cDNA duplexes were cloned into E. coli plasmid pBR322.

Although Example I below illustrates the cloning of a cDNA for a specific RNA segment (the L3 RNA species), this invention contemplates the use of any or all the segments from any BTV serotype depending on the specific use desired. For example, the probe generated from the L3 gene containing clone described in Example I reacts in a hybridization assay with all BTV serotypes tested including serotypes 10, 11, 15 and 17 common to the United States, BTV 1-15 common to South Africa, BTV-16 common to W. Pakistan and BTV-20 and -21 common to Australia. Thus, this probe is particularly valuable as a "group-specific" probe capable of detecting a wide variety of BTV serotypes.

Furthermore, albeit the clone described in Example I below represents a full-copy clone of the L3 gene segment of some 2772 nucleotide base pairs in length, it is clear that if it is desired, in its use as a source of probe DNA, the entire L3 gene segment would not be necessary. That is, a fragment of the L3 gene sequence would also be operable. It would be a matter of routine experimentation for one skilled in the art to fragment the L3 clone or any other

0178435

BTV-DNA containing clone, as, for example, by reaction with endonuncleases, to subclone the fragments and to test their ability to function as a group-specific or serotype-specific probe as described above. Thus, for the purpose of this invention the word probe as used herein means a nucleic acid molecule representing an entire gene segment or fragment thereof useful in the detection of viruses in an assay based on the hybridization of the probe with the nucleic acid of the virus.

In its use for the detection of BTV, the probes were labelled with an analytically detectable reagent. The invention, however, should not be limited to any particular means of detecting the probe-BTV hybrid. Example I employs a radioactive label for detection whereas Example II employs a non-radioactive label in the form of a biotin-avidin detection assay. Other detection methods are well-known in the art and may be easily substituted. Alternative systems, although not limiting would include fluorescent dyes, protein, immunological assays such as ELISA where antibodies to derivitized probe molecules are used, or antibodies to DNA-RNA hybrids themselves are used; competition assays wherein one strand of RNA is labelled with a inactive subunit of an enzyme and the probe is labelled with a second inactive subunit such that upon hybridization the subunits reassociate and enzyme activity is restored.

This invention is particularly useful for the detection of BTV in samples from infected cells or organisms employing the technique of _in situ_ hybridization. Samples which may be used comprise cell cultures, tissue samples such as brain or lung, biological fluids such as saliva, nasal secretion, urine, cervical or vaginal secretion, semen, pleural fluid, peritoneal fluid, serum, plasma, cerebrospinal fluid, middle ear fluid, joint fluid, gastric aspirate, or feces, and since BTV is known to be an arthropod-borne infection, the arthropod vector _Culicoides_ may also be monitored for the presence of the virus.

Because of the sensitivity of the assay system, the number of false negative determinations are reduced and because of the specificity of the hybridization, the number of false positive determinations are also reduced. Finally, because the component tested is the nucleic acid of the virus rather than the protein, the detrimental effect of mutation is lessened. That is, a single base change may result in elimination of an antigenic determinant when translated into protein whereas a single base pair mismatch would not affect the fidelity of a hybridization reaction.

This procedure has been successfully used for RNA recovered from infected cells or to identify viral RNA in cultures of cells using _in situ_ cytohybridization procedures. Irrespective of the method by which the viral genome can be detected using the cloned DNA probe, it is quite clear that the probe can provide a diagnostic tool for all BTV infections. By using the _in situ_ hybridization methods, numerous samples can be process simultaneously in a fairly short period of time. This is also sufficiently sensitive to be used with as little as a few micrograms of DNA.

This invention also contemplates a kit comprising a carrier being compartmented to receive a series of containers in close confinement which comprises a first container containing a DNA probe complementary to a portion of the RNA genome of a Bluetongue virus; a second container containing control non-Bluetongue virus RNA; and a third container containing control Bluetongue virus RNA.

The DNAs of the invention are also useful as a source of genetic information for the synthesis of BTV protein by means of recombinant DNA techniques. When correctly combined with an expression vector, so as to fall under the control of the promoter sequence of the vector, the vector containing the inserted viral DNA-segment may be used to transform a suitable host organism. The transformed host is then cultured under conditions promoting the synthesis of BTV protein.

The following Examples more fully illustrate the invention but should not be considered as limiting same.

0178435

## EXAMPLE I

This Example illustrates the cloning and sequencing of the L3 gene from BTV serotype 17.

Growth of virus and isolation of ds RNA: BTV serotype 17 was grown in BHK-21 cells and the double-stranded viral RNA was purified as described by Sugiyama et al., Amer. J. Epidermol 115: 332-347 (1981). The individual double-stranded segments were isolated by agarose gel electrophoresis as described by Rao et al., J. Virol 46: 378-381 (1983). Each RNA species was electroeluted from the gel as described by Maniatis et al. In: Molecular Cloning, Cold Spring Harbor Laboratory at pages 168-169 (1982) with the following modifications. Electroelution was carried out using only dialysis membranes to trap the eluted RNA. The RNA was collected by washing the membrane three times in 200 ul volumes of a solution containing 2mM Tris (pH 8.0), 2mM EDTA and 0.4M NaCl. Both BTV serotype 17 and BHK-21 cells are publically available from the American Type Culture Collection, 12301 Park Lawn Drive, Rockville, Maryland 20852-1976. The BTV strain has the accession number ATCC VR-875 whereas the well known Syrian or Golden Hamster Kidney cell line BHK-21 has the accession number ATCC CCL 10.

Polyadenylation of ds RNA: Preparations of ds RNA were denatured in the presence of 0.01M methyl mercury for 10 min at room temperature. Methyl mercury was then inactivated by addition of an equal amount of 2-mercaptoethanol. The 3' termini of both strands of ds RNA were polyadenylated using ATP and E. coli poly A polymerase (obtained from Bethesda Research Laboratories, Gaithersberg, MD) as described by Sippel, Eur. J. Biochem. 37: 31-40 (1973). The poly A tailed RNA was then purified through a Sephadex G-50 column and, prior to use for cDNA synthesis, again denatured with 10 mM methyl mercury.

Synthesis of cDNA: cDNA of the polyadenylated BTV RNA were synthesized using avian myeloblastosis virus reverse transcriptase (Life Sciences, St. Petersburg, FL) and 5'-phosphorylated oligo(dT)$_{12-18}$ (Collaborative Research, Waltham, MA) as primer (Maniatis et al. supra at pages 230-234). The reaction conditions were similar to those described by Buell et al. J. Biol. Chem. 253: 2471-2482 (1978). The RNA-cDNA hybrids were purified from nucleotides by a G-50 Sephadex column and precipitated with ethanol. The RNA templates were removed from RNA-DNA hybrids by incubating the hybrids in 0.3M KOH at room temperature overnight. After neutralization, the products were passed through a Sephadex G-50 column and cDNA precipitated with ethanol.

Preparation of full-length ds cDNA: The cDNA, which consisted of transcripts of both polarities, was dissolved in 50 ml of 10mM Tris-HCl (pH 8.0),0.4M NaCl, 1mM EDTA and self-annealed by incubation at 60°C for 24 hours. The annealed cDNA was collected by precipitation with ethanol. To ensure that the cDNA preparations were completely double-stranded, the products were treatd with the Klenow fragment of E. coli DNA polymerase I (New England Biolabs) prior to cloning as described by Bishop, D.H.L. et al. Nucleic Acid Res. 10: 1335-1343 (1982).

Molecular cloning: The E. coli plasmid was restricted with Hind III, the ends repaired with the Klenow fragment of E. coli DNA polymerase and dephosphorylated with alkaline phosphatase. The cDNA transcripts were blunt-end ligated to the plasmid using T$_4$ DNA ligase (New England Biolabs). By this means the Hind III site was reconstituted efficiently by the terminal A-T of the BTV cDNA. The hybrid plasmids were transfected into competent E. coli strain MC1061 cells (Casadaban MJ et al. J. Mol. Biol 138: 179-207 (1980) and colonies selected by resistance to ampicillin. The resulting transformants were screened for the presence of BTV-cDNA inserts by the Grunstein-Hogness colony hybridization assay (Grunstein, J. et al Proc. Nat'l Acad. Sci. USA 72: 3961-3965 (1978)) with a $^{32}$P-labeled cDNA short copy probe made using the oligo-dT primer and polyadenylated viral RNA (Bishop D.H.L. et al. supra). Hybridization positive colonies were characterized and sized by comparing their Hinf I restriction fragments to those of pBR322. The largest clones were subjected to "northern" blot and nucleotide sequence analysis.

RNA gel electrophoresis and blotting: RNA was extracted from BTV-17 infected BHK-21 cells and resolved by electrophoresis on 1.0% agarose gel as described previously. Following electrophoresis, the gels were prepared for blotting as described by Alwine et al. (Proc. Nat'l Acad. Sci. USA 74: 5350-5354 (1977)). Briefly, the gel was soaked in 50 mM NaOH, containing 14 mM mercaptoethanol and 1 ug/ml ethidium bromide for 20 minutes. The gel was washed 4 times (5 minutes each) in 25 mM sodium phosphate buffer pH 6.5, and osmotically blotted for 2 hours to a nitrocellulose hybridization transfer membrane (New England Nuclear, Boston, MA). After blotting, the membranes were air dried and baked in an oven at 80-100°C for at least 2 hours. Hybridization to DNA probes was performed by a procedure similar to that described by Denhardt (Biochem. Biophys. Res. Comm. 23: 641-652 (1966)). RNA blots were prehybridized at 42°C for 16 hours in 50% formamide containing 5 x SSC, 1% SDS, 100 ug/ml denatured salmon sperm DNA, and 0.04% each of polyvinyl-pyrrolidone, ficoll and bovine serum albumen (Sigma Chemical Company, St. Louis, MO). The membranes were then hybridized for 16 hours at 42°C to nick-translated,

$^{32}$P-labelled, DNA samples in the same formamide mixture. After hybridization, the membranes were washed twice at 25°C for 5 minutes 2 x SSC, twice at 65°C for 30 minutes each in 2 x SSC containing 0.5% SDS and twice for 30 minutes each at 25°C in 0.1 x SSC. After washing, the membranes were air dried and autoradiographed.

DNA sequence analyses. DNA nucleotide sequences were determined on strand separated, end-labeled, restriction fragments containing viral DNA insert sequences by the method of Maxam and Gilbert (Methods Enzmol. 65: 497-559 (1980)) using the formic acid protocol for the A+G reactions as described previously (Bishop, D.H.L. et al. supra).

Results

The size of one of the L gene clones (clone 7) was determined as shown in Figure 1. The DNA insert in the recombinant clone was excised with Hind III from plasmid DNA and run in a 1% agarose gel in parallel to molecular markers of DNA. The size of the DNA fragment, 2.8 Kb, suggested that it contained a full length L3 gene. By sequence analysis it was determined to be 2772 base pairs in length excluding the homopolymeric tails.

Since BTV cDNA L2 and L3 segments comigrated in agarose gels confirmation of the identity of the derived BTV DNA clone was obtained by determining the ds RNA genome segments to which they were capable of bybridizing. Figure 2A shows a northern blot of total BTV-17 RNA after electrophoresis in a 1% agarose gel and the Figure 28 demonstrates tht nick translated, $^{32}$P-labeled, probes from the clone hybridized specifically and only to the BTV-17 L3 RNA segment.

Nucleotide sequences and predicted gene products: The sequence of the cloned L3 gene was determined on strand-separated, end-labeled restriction DNA samples of one L3 specific clone (clone 7) by standard technique using the restriction endonuclease fragments as shown in Figure 3. The distance each DNA strand was sequenced is indicated by an unbroken arrow. The complete nucleotide sequence of DNA clone of L3 gene is presented in Figure 4. About 80% of the gene was analyzed in both directions. Excluding the homopolymeric tails, the entire sequence is 2772 nucleotides long. The L3 ds RNA has a corresponding size of $1.78 \times 10^6$ daltons in agreement with previous reports (Verwoerd, D.W. et al. J. Virology 10: 783-794 (1972)). A single open reading frame beginning at nucleotides 18-20 and ending at nucleotides 2721-2723, codes for a primary gene product composed of 901 amino acids with a calculated size of 103,412 daltons and -4 net charge.

0178435

TABLE I

AMINO ACID COMPOSITION OF BTV-12 SEGMENT 3 GENE PRODUCT

| Amino Acid | Residues |
|---|---|
| Alanine (A) | 65 |
| Arginine (R) | 69 |
| Aspartic (D) | 59 |
| Asparagine (N) | 42 |
| Cysteine (C) | 5 |
| Glutamic (E) | 50 |
| Glutamine (Q) | 42 |
| Glycine (G) | 42 |
| Histidine (H) | 16 |
| Isoleucine (I) | 65 |
| Leucine (I) | 82 |
| Lysine (K) | 28 |
| Methionine (M) | 37 |
| Phenylalanine (F) | 39 |
| Proline (P) | 46 |
| Serine (S) | 41 |
| Threonine (T) | 51 |
| Tryptophan (W) | 10 |
| Tyrosine (Y) | 39 |
| Valine (V) | 73 |
| | |
| Net charge | -4 |
| Mol. wt. | 103,412 |

Hybridization capability of clone L3 segment with equivalent segments of U.S. bluetongue virus and epizootic hemorrhagic disease virus (EHD), a related orbivirus: Since previous work has shown that the four U.S. serotypes, BTV-10,-11,-13 and -17 are genetically related to each other (e.g., they have comparable RNA fingerprints and can reassort their viral RNA species), the question of whether any sequence homologies between the L3 segments of these viruses was investigated. In order to detect any common sequences, a nick translated $^{32}$P-labelled cDNA probe of the L3 segment of BTV-17 was prepared and hybridized under stringent conditions to blots containing genomic RNAs purified from cells infected with either BTV-10 or -11 or -13 or -17, or EHD-1 virus. As shown in Figure 5, the $^{32}$P-labelled probe hybridized to the L3 RNA segments of all four U.S. serotypes, but did not hybridize to the equivalent segment of the related orbivirus, EHD. The results indicate that there are common nucleotide sequences in this gene for all U.S. bluetongue viruses.

Deposit with the A.T.C.C.

A deposit of the Clone L-3 transformed into E. coli was made with the American Type Culture Collection, 12301 Parklawn Drive, Rockville, Maryland on September 24, 1984, the accession number ATCC 39875 was assigned and the requisite fees were paid. Access to said culture will be available during pendency of this patent application to one determined by the Commissioner to be entitled thereto under 37 C.F.R. §1.14 and 35 U.S.C. §122. All restriction on availability of said culture to the public will be irrevocably removed upon the granting of a patent based upon this application and said culture will remain permanently available during the term of said patent, and should the

## EXAMPLE II

The Example illustrates the use of the BTV-17 L3 clone as a probe for the _in situ_ detection of BTV in infected cells.

Acid-cleaned coverslips are treated with denatured egg white and PVP (Brigati, D.J. _et al._ _Virology_ 126: 32-50 (1983)). These slips are sterilized by UV and placed in tissue culture dishes in which a monolayer of BHK-21 cells are grown overnight before a twenty hour infection by BTV. Some slips are not infected for use as negative controls.

Immediately following the infection, the slips are retrieved from the dishes, rinsed in PBS and fixed in Carnoy's B Fixative (60% nondenatured ethanol, 30% chloroform, 10% acetic acid) for five minutes at room temperature. After air drying, the slips can be stored for at least two weeks at room temperature of three months at -70°C.

The hybridization probe is prepared by incorporating biotinylated dUTP (Enzo Biochem) Inc. into the purified plasmid from the _E. coli_ strain containing pBR 322 with a cDNA copy of the L3 RNA from BTV17 by nick translation. This biotinylated probe was ethanol precipitated and dried before dissolving in hybridization solution containing 50% deionized formamide, 10% high molecular weight dextran sulfate, 2XSSC, and 0.4 mg/ml denatured salmon sperm DNA.

25ul drops of the above solution are placed on microscope slides and slips are placed cell-side down, sealed with rubber cement and steam heated to 80°C for five minutes. The slides are immediately placed on ice for three minutes and then left to hybridize at 37°C in a humid incubator for

0178435

at least four hours.  The slips are then removed from the slides and washed twice in 2XSSC at room temperature for 5 minutes, once in 2XSSC containing 0.1% SDS at 42°C for 20 minutes, and finally a 15 minute wash in PBS at room temperature.  Excess PBS is blotted from the slips after the final wash, and the slips are placed cell-side down on a drop of 0.1 mg/ml fluorescein conjugated avidin (Becton Dickinson) in PBS for 20 minutes at room temperature.  Finally, the slips are washed in PBS for 30 minutes at room temperature and observed under the fluorescent microscope.  Figure 6 illustrates photomicrographs of non-infected (6A) and BTV-infected (6B) cells.

0178435

## EXAMPLE III

This Example illustrates the reactivity of the BTV 17 L3 clone with a variety of BTV serotypes.

In order to detect whether cloned DNA of segment 3 of BTV-17 share any homologous gene sequences with other BTV serotypes originally isolated from South Africa and Australia, the norther blot hybridization technique was employed. Purified double stranded RNAs of each bluetongue virus serotype was electrophoresed and blotted on Genescreen (NEN, Boston, MA) as described by Purdy, M. et al., J. of Virol. 51(3):754-759(1984). Figure 7 shows the electrophoretic patterns of the ten RNA segments of 19 BTV serotypes. As had been noted previously for four U.S. serotypes (BTV-10, -11, -13 and -17), the 19 different viruses exhibit strikingly similar pattern of RNA segments in this gel system. Figure 8 shows the results of hybridizing the blotted viral RNA segments to $^{32}$P-labelled nick-translated DNA of segment 3 of BTV-17. As shown in Figure 8, RNA segment 3 of each serotype hybridized to the DNA.

Initially experiments to detect viral gene sequences in infected cell culture employed nick translated $^{32}$P-labelled DNA probe. Hamster lung (HmLu) cells were grown on 4-8 compartment slides (Miles Laboratories) and infected at a multiplicity of 0.1-1PFU/cell with each BTV serotype. At 16-20 hours post-infection, slides containing infected cell cultures were removed from the growth medium and rinsed 3 times with phosphate buffered saline (PBS). The cells were fixed in Carony's B fixative (60% ethanol, 30% chloroform, 10% acetic acid) for 5 minutes according to the instruction manual of Enzo Bio-Chem. Inc. (1983). The samples were then drained, air-dried and stored at 4°C. Each chamber

0178435

containing dehydrated cells were overlaid with 25-50ul of the hybridization mixture containing the $^{32}$P-DNA probe. The lid of the chamber slide was then sealed with tape, placed in a container, and heated at 80°C in a bath for 5 minutes, as described by Brigatti et al. (supra). The slides were then transferred to a 37°C incubator for 12-16 hours. After hybridization the solutions were removed and kept at 4°C for re-use. The chambers and plastic liners were removed from the chamber slides and each slide was washed in 2xSSC and PBS as suggested by the Enzo Bio-Chem. Inc. manuals. For detecting hybridized DNA containing radioactivity, the slides were simply dried and autodiographed. Figure 9 illustrates the results. In some well the cells detached prior to 16 hours (e.g. BTV-2, BTV-6) and the analysis was repeated using a lower multiplicity of infection. (0.01-0.1 Pfu per cell).

The presence of viral infection was detected by this procedure in each of the monolayers containing infected cells; none were detected in the control, uninfected cells.

Similar hybridization experiments were performed using a nick-translated, biotin-labelled, DNA probe instead of $^{32}$P-labelled probe. For the fluorescence detection of hybridized DNA containing biotin, the probe was removed, slides were washed, air-dried similarly as above and then 25-50ul of fluorescein-conjugated avidin (0.1mg/ml, purchased from Becton-Dickinson) was added to each chamber. The samples were incubated at 18°C for 30 minutes, then washed three times in PBA for 5 minutes, blot-dried, mounted with glycerol and examined for fluorescence. Figure 10 shows a typical result of hybrids identified following incubation with the conjugated avidin-fluorescein complex. Again, strong fluorescent signals were observed with the infected cells, and in contrast none were observed with the control cells.

To obtain some measure of the relative sensitivity of the in situ hybridization system HmLu cells were infected with BTV-2 in different m.o.i. of the virus. Infected chamber slides were fixed at different time intervals post-infection and were processed for in situ hybridization with nick translated $^{32}$P-DNA probes. The genome can be detected as early as 8 hours of post-infection, and it was clear that the nucleic acid hybridization is directly proportional to the input multiplicity of infection of the virus to the cells. These results indicate that reasonably high titers of virus can be detected quite rapidly by this procedure.

0178435

85110967.8

What is Claimed is:

1. A DNA sequence characterized in that it is capable of hybridizing with the RNA genome of at least one serotype of Bluetongue virus.

2. The sequence according to Claim 1 wherein said serotype is BTV-17 and said sequence is substantially the following in a 5' to 3' direction

GT TAA ATT TCC GTA GCC ATG GCT GCT CAG AAT GAG CAA CGT CCG
GAA CGA ATA AAG ACG ACG CCG TAT TTA GAA GGG GAT GTG TTA TCA
AGC GAC TCA GGG CCA CTG CCT TCC GTG TTC GCG TTG CAA GAG ATA
ATG CAA AAG GTG AGG CAA GTG CAA GCT GAT TAT ATG ACA GCA ACA
CGA GAG GTT GAT TTT ACA GTA CCG GAT GTA CAA AAG ATT CTT GAT
GAC ATT AAA ACG TTA GCT GCA GAA CAA GTG TAC AAA ATC GTC AAA
GTA CCT AAT ATT TCA TTC AGA CAT ATC GTA ATG CAG TCA AGA GAT
CGA GTT TTA CGA GTG GAT ACC TAC TAC GAA GAG ATG TCA CAG GTT
GGA GAT GTT ATA ACG GAA GAT GAA CCA GAA AAA TTC TAT TCA ACT
ATA ATC AAG AAA GTG CGG TTT ATA CGC GGA AAA GGA TCC TTT ATA
TTA CAT GAT ATT CCG ACC AGA GAT CAT CGC GGC ATG GAG GTT GCT
GAG CCA GAA GTG TTA GGA GTC GAA TTC AAG AAT GTA CTA CCT GTG
TTG ACA GCC GAG CAT CGC GCA ATG ATT CAG AAC GCA TTG GAT GGA
TCG ATA ATT GAG AAC GGA AAC GTA GCT ACA CGA GAC GTT GAC GTA
TTC ATA GGC GCC TGT TCG AAC CCA ATC TAT CGC ATA TAC AAT AGA
GTG CAA GGG TAT ATT GAG GCA GTG CAA TTA CAA GAG TTA AGG AAT
TCA ATT GGG TGG TTA GAG AGG TTA GGG CAG AGG AAA AGA ATC ACG
TAT TCG CAA GAG GTT CTG ACT GAT TTT AGG AGG CAA GAC ACA ATT
TGG GTC TTA GCT TTA CAG CTA CCG GTC AAT CCA CAG GTA GTG TGG

```
GAT GTG CCG CGC AGC TCT ATC GCC AAC TTA ATC ATG AAT ATA GCA
ACG TGC TTA CCC ACA GGG GAA TAC ATC GCG CAA AAC CAA ACA ATT
TCA TCG ATT ACG CTG ACC CAA AGA ATA ACA ACA ACG GGG CCA TTT
GCT ATT CTA ACT GGA TCA ACC CCA ACT GCA CAG CAA CTT AAT GAT
GTT AGG AAG ATC TAT TTA GCG CTA ATG TTT CCT GGA CAG ATT ATA
CTT GAT CTA AAA ATC GAT CCT GGC GAG AGG ATG GAT CCG GCA GTA
AGA ATG CTC GCT GGC GTT GTA GGT CAT TTG CTC TTT ACA GCG GGT
GGA AGA TTC ACG AAT TTA ACA CCA AAT ATG GCG AGA CAG CTC GAT
ATA GCC CTA AAC GAT TAT TTA CTT TAT ATG TAT AAC ACC AGA GTT
CAA GTC AAT TAT GGT CCA ACG GGT GAG CCG TTA GAT TTC CAG ATT
GGA AGG AAT CAG TAT GAC TGT AAT GTT TTT AGA GCA GAT TTC GCG
ACA GGA ACA GGA TAC AAC GGA TGG GCT ACA ATT GAT GTT GAG TAT
AGA GAC CTG GCC CCT TAC GTG CAT GCG CAG CGC TAC ATA CGT TAT
TGT GGT ATC GAT TCG CGC GAG TTG ATT AAT CCG ACA ACA TAT GGC
ATT GGG ATG ACT TAT CAT TGT TAC AAT GAG ATG TTA CGA ATG TTA
GTT GCC GCA GGG AAA GAT TCT GAG GCG GCG TAC TTT CGC AGC ATG
CTG CCC TTT CAC ATG GTA AAG TTT GCT AGG ATA AAT CAA ATC ATA
AAC GAA GAT TTA CAC TCT GTG TTC TCG TTG CCG GAT CAT ATG TTC
AAC GCA TTA TTA CCC GAC CTA ATT GCT GGG GCG CAT CAG AAC GCC
GAC CCA GTT GTG CTA GAT GTG AGT TGG ATA TCG CTG TGG TTC GCT
TTC AAC AGG TCG TTT GAA CCA ACG CAT AGG AAT GAG ATG CTC GAA
ATC GCT CCA CTG ATC GAG TCC GTT TAT GCG TCG GAG CTT TCT GTG
ATG AAG GTA GAT ATG CGA CAC TTG TCA TTA ATG CAG AGA AGA TTC
CCA GAT GTT TTA ATC CAA GCG AGG CCG TCC CAT TTT TGG AAA GCA
GTT CTG AAT GAC AGC CCG GAG GCG GTG AAA GCA GTT ATG AAC TTA
TCG CAT TCG CAT AAT TTC ATC AAT ATA AGG GAT ATG ATG CGT TGG
GTA ATG CTC CCA TCA CTG CAA CCA TCG TTA AAG CTC GTA TTA GAA
GAG GAG GCA TGG GCC GCT GCA AAC GAT TTC GAA GAT CTG ATG CTT
ACT GAT CAA GTT TAT ATG CAT CGA GAT ATG TTG CCA GAA CCA CGG
TTG GAT GAT GTT GAG AGG TTC AGA CAG GAA GGT TTC TAT TAC ACG
AAC ATG TTA GAG GCC CCA CCA GAA ATA GAT CGT GTA GTT CAG TAT
ACT TAT GAG ATT GCA CGT TTA CAA GCG AAC ATG GGA CAA TTT CGG
```

GCA GCT CTA AGA CGC ATT ATG GAT GAT GAT GAC TGG GTG AGA TTT
GGC GGT GTC TTA CGC ACT GTA CGC GTT AAA TTC TTT GAT GCG CGA
CCC CCA GAC GAT ATT CTA CAG GGC TTA CCT TTC AGC TAT GAT ACA
AAC GAG AAA GGT GGA TTA TCA TAT GCG ACG ATT AAG TAT GCT ACT
GAG ACC ACA ATT TTT TAT CTG ATA TAT AAT GTC GAA TTC TCG AAC
ACG CCT GAT TCT TTG GTG TTG ATT AAT CCA GCA TAC ACG ATG ACT
AAA GTT TTC ATT AAC AAG AGA ATT GTT GAG CGA GTA CGG GTT AGA
CAA ATT TTG GCC GTA TTG AAC AGA AGA TTC GTG GCA TAC AAA GGA
AAA ATG AGA ATT ATG GAC ATC ACT CAA TCG CTC AAG ATG GGC GCC
AAG CTG GCT GCG CCA ACT GTG TAG ATG CGC GAC CAA TCT ATG CAC
TTG GTA GCG GCA GCG GAA ATA CAC TTA C

3.    A cloning vector characterized in that it comprises the DNA sequence of Claims 1 or 2.

4.    A host organism transformed by the vector of Claim 3.

5.    The host according to Claim 4 wherein the host has the identifying characteristics of ATCC 39875.

6.    A method for producing a duplex DNA sequence that is capable of hybridizing with the RNA genome of at least one serotype of Bluetongue virus which comprises

a)    isolating a double-stranded RNA species from a Bluetongue virus serotype;

b)    separating the RNA strands;

c)    polyadenylating the 3' ends of each RNA strand to provide RNA template strands

d)    synthesizing a cDNA copy of each RNA template strand by contacting said polyadenylated RNA template strands with a reverse transcriptase in the presence of oligo dT under conditions of time and temperature sufficient for the production of cDNA;

e)   removing the RNA strands;  and

f)   self-annealing the cDNA products to form cDNA duplexes.

7.   A DNA probe useful for the detection of Bluetongue virus characterized as comprising a DNA sequence complementary to a portion of the RNA genome of said virus and capable of hybridization therewith.

8.   A method for detection of Bluetongue virus in a sample containing same comprising:

a)   providing an analytically detectable probe DNA complementary to a portion of the RNA genome of said virus.

b)   contacting said probe with said RNA under condition promoting the formation of DNA-RNA hybrids and

c)   detecting the DNA-RNA hybrids, thereby detecting the presence of the virus in said sample.

9.   The method according to Claim 8 wherein step (b) is performed in situ.

10.   A kit comprising a carrier being compartmented to receive a series of containers in close confinement which comprises a first container containing a DNA probe complementary to a portion of the RNA genome of a Bluetongue virus;

a second container containing control non-Bluetongue virus RNA;  and

a third container containing control Bluetongue virus RNA.

11.   A kit according to Claim 10 wherein said probe comprises the sequence of Claims 1 or 2.

Fig. 1

Fig. 2

85110967.8

0178435

Fig. 3

```
CT TAA ATT TCC GTA GCC ATG GCT GCT CAG AAT GAG CAA CGT CCG
GAA CGA ATA AAG ACG ACG CCG TAT TTA GAA GGG GAT GTG TTA TCA
AGC GAC TCA GGG CCA CTG CCT TCC GTG TTC GCG TTG CAA GAG ATA
ATG CAA AAG GTG AGG CAA GTG CAA GCT GAT TAT ATG ACA GCA ACA
CGA GAG GTT GAT TTT ACA GTA CCG GAT GTA CAA AAG ATT CTT GAT
GAC ATT AAA ACG TTA GCT GCA GAA CAA GTG TAC AAA ATC GTC AAA
GTA CCT AAT ATT TCA TTC AGA CAT ATC GTA ATG CAG TCA AGA GAT
CGA GTT TTA CGA GTG GAT ACC TAC TAC GAA GAG ATG TCA CAG GTT
GGA GAT GTT ATA ACG GAA GAT GAA CCA GAA AAA TTC TAT TCA ACT
ATA ATC AAG AAA GTG CGG TTT ATA CGC GGA AAA GGA TCC TTT ATA
TTA CAT GAT ATT CCG ACC AGA GAT CAT CGC GGC ATG GAG GTT GCT
GAG CCA GAA GTG TTA GGA GTC GAA TTC AAG AAT GTA CTA CCT GTG
TTG ACA GCC GAG CAT CGC GCA ATG ATT CAG AAC GCA TTG GAT GGA
TCG ATA ATT GAG AAC GGA AAC GTA GCT ACA CGA GAC GTT GAC GTA
TTC ATA GGC GCC TGT TCG GAA CCA ATC TAT CGC ATA TAC AAT AGA
GTG CAA GGG TAT ATT GAG GCA GTG CAA TTA CAA GAG TTA AGG AAT
TCA ATT GGG TGG TTA GAG AGG TTA GGG CAG AGG AAA AGA ATC ACG
TAT TCG CAA GAG GTT CTG ACT GAT TTT AGG AGG CAA GAC ACA ATT
TGG GTC TTA GCT TTA CAG CTA CCG GTC AAT CCA CAG GTA GTG TGG
GAT GTG CCG CGC AGC TCT ATC GCC AAC TTA ATC ATG AAT ATA GCA
ACG TGC TTA CCC ACA GGG GAA TAC ATC GCG CAA AAC CAA AGA ATT
TCA TCG ATT ACG CTG ACC CAA AGA ATA ACA ACA ACG GGG CCA TTT
GCT ATT CTA ACT GGA TCA ACC CCA ACT GCA CAG CAA CTT AAT GAT
GTT AGG AAG ATC TAT TTA GCG CTA ATG TTT CCT GGA CAG ATT ATA
CTT GAT CTA AAA ATC GAT CCT  GGC GAG AGG ATG GAT CCG GCA GTA
AGA ATG GTC GCT GGC GTT GTA GGT CAT TTG CTC TTT ACA GCG GGT
GGA AGA TTC ACG AAT TTA ACA CCA AAT ATG GCG AGA CAG CTC GAT
ATA GCC CTA AAC GAT TAT TTA CTT TAT ATG TAT AAC ACC AGA GTT
CAA GTC AAT TAT GGT CCA ACG GGT GAG CCG TTA GAT TTC CAG ATT
GGA AGG AAT CAG TAT GAC TGT AAT GTT TTT AGA GCA GAT TTC GCG
ACA GGA ACA GGA TAC AAC GGA TGG GCT ACA ATT GAT GTT GAG TAT
```

Fig. 4 (a)

AGA GAC CTG GCC CCT TAC GTG CAT GCG CAG CGC TAC ATA CGT TAT
TGT GGT ATC GAT TCG CGC GAG TTG ATT AAT CCG ACA ACA TAT GGC
ATT GGG ATG ACT TAT CAT TGT TAC AAT GAG ATG TTA CGA ATG TTA
GTT GCC GCA GGG AAA GAT TCT GAG GCG GCG TAC TTT CGC AGC ATG
CTG CCC TTT CAC ATG GTA AAG TTT GCT AGG ATA AAT CAA ATC ATA
AAC GAA GAT TTA CAC TCT GTG TTC TCG TTG CCG GAT CAT ATG TTC
AAC GCA TTA TTA CCC GAC CTA ATT GCT GGG GCG CAT CAG AAC GCC
GAC CCA GTT GTG CTA GAT GTG AGT TGG ATA TCG CTG TGG TTC GCT
TTC AAC AGG TCG TTT GAA CCA ACG CAT AGG AAT GAG ATG CTC GAA
ATC GCT CCA CTG ATC GAG TCC GTT TAT GCG TCG GAG CTT TCT GTG
ATG AAG GTA GAT ATG CGA CAC TTG TCA TTA ATG CAG AGA AGA TTC
CCA GAT GTT TTA ATC CAA GCG AGG CCG TCC CAT TTT TGG AAA GCA
GTT CTG AAT GAC AGC CCG GAG GCG GTG AAA GCA GTT ATG AAC TTA
TCG CAT TCG CAT AAT TTC ATC AAT ATA AGG GAT ATG ATG CGT TGG
GTA ATG CTC CCA TCA CTG CAA CCA TCG TTA AAG CTC GTA TTA GAA
GAG GAG GCA TGG GCC GCT GCA AAC GAT TTC GAA GAT CTG ATG CTT
ACT GAT CAA GTT TAT ATG CAT CGA GAT ATG TTG CCA GAA CCA CGG
TTG GAT GAT GTT GAG AGG TTC AGA CAG GAA GGT TTC TAT TAC ACG
AAC ATG TTA GAG GCC CCA CCA GAA ATA GAT CGT GTA GTT CAG TAT
ACT TAT GAG ATT GCA CGT TTA CAA GCG AAC ATG GGA CAA TTT CGG
GCA GCT CTA AGA CGC ATT ATG GAT GAT GAT GAC TGG GTG AGA TTT
GGC GGT GTC TTA CGC ACT GTA CGC GTT AAA TTC TTT GAT GCG CGA
CCC CCA GAC GAT ATT CTA CAG GGC TTA CCT TTC AGC TAT GAT ACA
AAC GAG AAA GGT GGA TTA TCA TAT GCG ACG ATT AAG TAT GCT ACT
GAG ACC ACA ATT TTT TAT CTG ATA TAT AAT GTC GAA TTC TCG AAC
ACG CCT GAT TCT TTG GTG TTG ATT AAT CCA GCA TAC ACG ATG ACT
AAA GTT TTC ATT AAC AAG AGA ATT GTT GAG CGA GTA CGG GTT AGA
CAA ATT TTG GCC GTA TTG AAC AGA AGA TTC GTG GCA TAC AAA GGA
AAA ATG AGA ATT ATG GAC ATC ACT CAA TCG CTC AAG ATG GGC GCC
AAG CTG GCT GCG CCA ACT GTG TAG ATG CGC GAC CAA TCT ATG CAC
TTG GTA GCG GCA GCG GAA ATA CAC TTA C

Fig. 4 (b)

Fig. 5

85110967.8

0178435

Fig. 6a

Fig. 6b

Fig 7.

Fig. 8

0178435

BTV serotype infected or control cells

hybridized to BTV-17 segment #3 DNA

Fig. 9

Fig. 10